# EUROPÄISCHE PATENTSCHRIFT

(11) **EP 2 172 233 B1**
(45) Veröffentlichungstag und Bekanntmachung des Hinweises auf die Patenterteilung: **19.10.2016**
(21) Anmeldenummer: 09169482.8
(22) Anmeldetag: 04.09.2009
(51) Int. Cl.: A61L 31/02, A61L 31/14

(54) **Implantat mit einem Grundkörper aus einer biokorrodierbaren Manganlegierung**
Implant with a base body made of a bio-corrodible manganese alloy
Implant doté d'un corps de base constitué d'un alliage de manganèse biocorrodable

(30) Priorität: 02.10.2008 DE 102008042578
(43) Veröffentlichungstag der Anmeldung: 07.04.2010
(73) Patentinhaber: Biotronik VI Patent AG, 6341 Baar (CH)
(72) Erfinder: Müller, Heinz, 91052, Erlangen (DE)
(74) Vertreter: Galander, Marcus

(56) Entgegenhaltungen:
- EP-A2- 2 289 575
- WO-A1-01/21229
- DE-A1- 19 716 795
- US-A1- 2004 129 347
- H. Hermawan ET AL: "Iron-manganese: new class of metallic degradable biomaterials prepared by powder metallurgy", Powder Metallurgy, vol. 51, no. 1, 1 March 2008 (2008-03-01), pages 38-45, XP055191814, ISSN: 0032-5899, DOI: 10.1179/174329008X284868

## Beschreibung

Die Erfindung betrifft ein Implantat mit einem Grundkörper, der ganz oder in Teilen aus einer biokorrodierbaren Manganlegierung besteht.

Implantate finden in vielfältiger Ausführungsform in der modernen Medizintechnik Anwendung. Sie dienen unter anderem der Unterstützung von Gefäßen, Hohlorganen und Gangsystemen (endovaskuläre Implantate), zur Befestigung und temporären Fixierung von Gewebeimplantaten und Gewebstransplantationen, aber auch zu orthopädischen Zwecken, zum Beispiel als Nagel, Platte oder Schraube.

Die Implantation von Stents ist eine der wirkungsvollsten therapeutischen Maßnahmen bei der Behandlung von Gefäßerkrankungen. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Der Grundkörper jedes Implantats, insbesondere von Stents, besteht aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßem Zweck mit der Körperumgebung in Kontakt steht, ist dessen Körperverträglichkeit (Biokompatibilität). Unter Biokompatibilität wird die Fähigkeit eines Werkstoffs verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantats an das Empfängergewebe mit dem Ziel einer klinisch erwünschten Wechselwirkung. Die Biokompatibilität des Implantatwerkstoffs ist weiterhin abhängig vom zeitlichen Ablauf der Reaktion des Biosystems in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatswerkstoffs in verschiedener Weise. Entsprechend der Reaktion des Biosystems können die Implantatwerkstoffe in bioaktive, bioinerte und degradierbare/resorbierbare Werkstoffe unterteilt werden. Für die Zwecke der vorliegenden Erfindung sind lediglich degradierbare/resorbierbare, metallische Implantatwerkstoffe von Interesse, die nachfolgend als biokorrodierbare metallische Werkstoffe bezeichnet werden.

Der Einsatz biokorrodierbarer metallischer Werkstoffe bietet sich insbesondere schon deshalb an, da häufig nur ein zeitweiliger Verbleib des Implantats im Körper zur Erfüllung des medizinischen Zweckes erforderlich ist. Implantate aus permanenten Werkstoffen, also Werkstoffen, die im Körper nicht abgebaut werden, sind gegebenenfalls wieder zu entfernen, da es mittel- und langfristig auch bei hoher Biokompatibilität zu Abstoßungsreaktionen des Körpers kommen kann.

Ein Ansatz zur Vermeidung eines weiteren chirurgischen Eingriffs besteht demnach darin, das Implantat ganz oder in Teilen aus einem biokorrodierbaren metallischen Werkstoff zu formen. Unter Biokorrosion werden Vorgänge verstanden, die durch die Anwesenheit von Körpermedien bedingt sind und die zu einem allmählichen Abbau der aus dem Werkstoff bestehen Struktur führen. Zu einem bestimmten Zeitpunkt verliert das Implantat oder zumindest der Teil des Implantates, der aus dem biokorrodierbaren Werkstoff besteht, seine mechanische Integrität. Die Abbauprodukte werden vom Körper weitgehend resorbiert. Die Abbauprodukte haben, wie beispielsweise beim Magnesium, im günstigsten Fall sogar eine positive therapeutische Wirkung auf das umgebende Gewebe. Geringe Mengen nicht resorbierbarer Legierungsbestandteile sind - sofern sie nicht toxisch sind - tolerierbar.

Bekannte biokorrodierbare metallische Werkstoffe umfassen Reineisen und biokorrodierbare Legierungen der Hauptelemente Magnesium, Eisen, Zink, Molybdän und Wolfram. In DE 197 31 021 A1 wird unter anderem vorgeschlagen, medizinische Implantate aus einem metallischen Werkstoff zu formen, dessen Hauptbestandteil ein Element aus der Gruppe Alkalimetalle, Erdalkalimetalle, Eisen, Zink und Aluminium ist. Als besonders geeignet werden Legierungen auf Basis von Magnesium, Eisen und Zink beschrieben. Nebenbestandteile der Legierungen können Mangan, Kobalt, Nickel, Chrom, Kupfer, Cadmium, Blei, Zinn, Thorium, Zirkonium, Silber, Gold, Palladium, Platin, Silizium, Calcium, Lithium, Aluminium, Zink und Eisen sein. Ungeachtet der erreichten Fortschritte auf dem Gebiet biokorrodierbarer Metalllegierungen sind die bisher bekannten Legierungen aufgrund ihres Korrosionsverhaltens nur beschränkt einsatzfähig.

So zeigte sich, dass metallische Implantate aus Magnesium-Legierungen *in vivo* bedingt durch das rasche Auflösen bereits nach wenigen Tagen ihre Stützkraft verlieren.

Auch für Reineisen oder bekannte Eisenlegierungen sind die Einsatzmöglichkeiten aufgrund der relativ langsamen Biokorrosion limitiert.

Um die Korrosion von Reineisen oder Eisenlegierungen unter physiologischen Bedingungen zu erhöhen, wurden verschiedene Legierungselemente getestet. So ist hierzu beispielsweise Mangan geeignet, das üblicherweise als Legierungselement für hoch verschleißbeständige Stähle, so genannte Mn-Hartstähle, bekannt ist und dort in Mengen von ca. 12 bis ca. 25 Gew.% verwendet wird. Allerdings neigen diese Legierungen, unter den üblichen Anwendungsbedingungen, im Allgemeinen nur wenig zu Korrosion. Hermawan et al "Iron-manganese: new class of metallic degradable biomaterials prepared by powder metallurgy" Powder Metallurgy (2008), Vol. 51, No. 1 offenbart eine Fe - 35 Gew% Mn - Legierung mit dem Ziel der Verwendung als metallischer, degradierbarer Werkstoff eines Stents.

DE 197 16 795 A1 offenbart eine hochfeste und korrosionsbeständige Eisen-Mangan-Chrom-Legierung, und die Verwendung dieser Legierung zur Herstellung von temporären Implantaten.

Der vorliegenden Erfindung liegt daher die Aufgabe zugrunde, einen hinsichtlich seines Korrosionsverhaltens verbesserten biokorrodierbaren metallischen Werkstoff für ein Implantat bereitzustellen. Dies soll insbesondere derart erfolgen, dass die weiteren, für die Bearbeitung wichtigen Materialeigenschaften, wie zum Beispiel seine Duktilität und Festigkeit, sich unwesentlich verändern und gegebenenfalls sogar noch verbessern.

Die Aufgabe wird erfindungsgemäß durch ein medizinisches Implantat gelöst, dessen Grundkörper ganz oder in Teilen aus einer Legierung besteht, deren Hauptbestandteil Mangan ist (Mangan-Basislegierung).

Die erfindungsgemäße Legierung enthält Mangan als Hauptkomponente. Unter Hauptkomponente einer Legierung wird erfindungsgemäß die Legierungskomponente verstanden, deren Atomanteil an der Legierung am höchsten ist. In den nachfolgenden noch näher beschriebenen bevorzugten Legierungszusammensetzungen sind alle weiteren Anteile der Legierungskomponenten immer so vorzugeben, dass jeweils Mangan einschließlich herstellungsbedingter Verunreinigungen den höchsten Atomanteil hat. Dementsprechend verstehen sich alle prozentualen Angaben zur Zusammensetzung der Legierung als Angaben in Atomprozent.

Elementares Mangan ist ein stahl-weißes und hartes Schwermetall, das in vier verschiedenen Modifikationen mit unterschiedlichem kristallographischem Aufbau existiert (α-, β-, γ-, δ-Mangan), die beim Erhitzen auf 740°C, 1075°C und auf über 1140°C zugänglich sind. So kristallisiert Mangan bei Temperaturen unter 720°C als α-Mn, der bei Raumtemperatur beständigen und sehr spröde Modifikation des Elementes, welches als kubischraumzentriertes Metallgitter (krz-Phase, α-Phase) vorliegt.

Besonders bevorzugte Mangan-Basislegierungen im Rahmen der vorliegenden Erfindung sind γ-Manganlegierungen. Diese zeichnen durch die Ausbildung eines kubischflächenzentrierten (kfz) Metallgitters aus, einer sogenannten γ-Phase oder austenitischen Phase.

Der Begriff Austenit wird sowohl zur Kennzeichnung von kfz-Stählen, z.B. der handelsüblichen CrNi-Stähle, als auch für kubisch-flächenzentrierte Modifikationen anderer Legierungen, wie z.B. beim System Ni-Ti, verwendet.

Ein Austenit im Rahmen der vorliegenden Erfindung ist ein kubisch-flächenzentrierter Mischkristall einer Mangan-Basislegierung, bei dem die beim Rein-Mangan bei Raumtemperatur vorliegende kubisch-raumzentrierte Phase durch ein oder mehrere Legierungselemente oder durch die Kombination eines oder mehrerer Legierungselemente und einer thermischen Behandlung aus dem γ-Feld, unterdrückt wird. Dies kann einerseits durch bestimmte Konzentrationen geeigneter Legierungselemente allein erfolgen, durch welche die Umwandlung aus dem γ-Gebiet beim Abkühlen bei der schmelzmetallurgischen Herstellung der Legierungen unterbleibt. Zum anderen kann dies dadurch eintreten, dass eine Legierung mit einem Element, das die Umwandlung erschwert, d.h. zu tieferen Temperaturen verlagert, diese aber nicht komplett unterdrücken kann, aus dem γ-Gebiet auf Raumtemperatur abgeschreckt wird und dort dann als metastabile γ-Phase vorliegt. Geeignete Legierungselemente im obigen Sinne sind z.B.: C, N, Pd, Pt, Rh, Ru, V, Ir, Cu, Fe und Au.

Diese austenitischen Legierungen sind paramagnetisch und ermöglichen je nach Wahl des oder der weiteren Legierungselemente eine bessere Umformbarkeit und Duktilität als das spröde α- oder β-Mangan.

Für den Fall, dass die γ-Phase der Mangan-Basislegierung bei Raumtemperatur nicht stabil ist, sind dem Fachmann thermochemische Verfahren bekannt, beispielsweise durch die Einstellung der Abkühlgeschwindigkeit bei der Herstellung der Legierung, die Umwandlung der kfz-Phase in die α- oder β-Phase zu unterdrücken, sodass ein rein austenitisches Gefüge vorliegt. Dabei wird, durch das schnelle Abkühlen, die Temperatur so weit abgesenkt, dass die für die Phasenumwandlung erforderliche Diffusion nicht mehr stattfinden kann und die γ-Phase bei Raumtemperatur metastabil eingefroren wird.

Besonders gute Korrosionseigenschaften wurden für Manganlegierungen mit Eisen und/oder Iridium als weiteres Legierungselement beobachtet.

Für einen biologischen Abbau besonders günstige Korrosionseigenschaften zeigen Mangan-Eisen-Legierungen ab einem Anteil von mehr als 50% Mangan.

Besonders bevorzugte beträgt der Anteil von Eisen an der erfindungsgemäßen Manganlegierung 40 bis kleiner 50%. Bei diesem Anteil von Eisen ist das austenitische Gefüge der Manganlegierungen bis auf Raumtemperatur thermodynamisch stabil.

Weiterhin bevorzugt beträgt der Anteil von Eisen 5 bis 40%. In Versuchen wurde insbesondere bei einer Zulegierung von 10 bis 30% Eisen eine erhebliche Verringerung der Korrosionsbeständigkeit gemessen.

Es hat sich gezeigt, dass Manganlegierungen mit Eisen, insbesondere γ-Manganlegierungen, bei Verformung sehr stark verfestigen und extrem verschleißbeständig sind.

Vorteilhafte Abbauzeiten ergeben sich weiter bei Werkstoffen mit dem Hauptbestandteil Mangan und bis zu 25% Iridium.

Besonders bevorzugt beträgt der Anteil von Iridium an der erfindungsgemäßen Manganlegierung zwischen 8 bis 15%. Diese Manganlegierungen zeigen eine erheblich bessere Duktilität als die spröde α-Phase des Mangans und eine hohe Festigkeit. Auch in diesem Legierungsbereich ist die γ-Phase des Mangans bis auf Raumtemperatur thermodynamisch stabil.

Bei höheren oder geringeren Legierungsgehalten an Iridium muss der Werkstoff zum Erhalt eines bei Raumtemperatur stabilen kfz-Gitters einer thermischen Behandlung unterworfen werden.

Werden sowohl Eisen als auch Iridium reinem Mangan zulegiert, so kann ebenfalls die Korrosionsbeständigkeit verringert werden. In Versuchen wurde z.B. insbesondere bei einer Zulegierung zwischen 30 bis 40% Eisen mit einem Iridium-Gehalt bis zu 10% bzw. zwischen 10 bis 20% Eisen und mit einem Iridium-Gehalt zwischen 8 bis 20% eine erhebliche Verringerung der Korrosionsbeständigkeit gemessen.

Tabelle 1 umfasst die im ternären System Mn-Fe-Ir-Legierung besonders bevorzugten Legierungsbereiche. Die Angaben der Anteile an Eisen und Iridium in den jeweiligen Formeln (I) bis (IV) sind in Atomprozent zu verstehen.

**Tabelle 1**

| **Mn - Fe - Ir-Legierung** | | |
|---|---|---|
| Mn - Fe_{(50-3.57X)}-Ir_{X} | mit X = 0.1 - 13.99 | Formel (I) |
| Mn - Fe_{(40-5.0X)} - Ir_{X} | mit X = 0.1 - 7.99 | Formel (II) |
| Mn - Fe_{(50-2.0X)}-Ir_{X} | mit X = 0.1 - 24.99 | Formel (III) |
| Mn - Fe_{(20-10.0X)}-Ir_{X} | mit X = 0.1 - 1.99 | Formel (IV) |

Die biologischen, mechanischen und chemischen Eigenschaften der erfindungsgemäßen Werkstoffe können weiterhin positiv beeinflusst werden, wenn ein oder mehrere Elemente ausgewählt aus der Gruppe bestehend aus Kupfer (0 - 20 At.%), Gold (0 - 20 At.%), Palladium (0 - 20 At.%), Platin (0 - 20 At.%), Rhenium(0 - 20 At.%), Ruthenium (0 - 10 At.%), Vanadium (0 - 10 At.%), Kohlenstoff (0 - 5 At.%), Stickstoff (0 - 5 At.%), Arsen (0 - 5 At.%) und Selen (0 - 5 At.%) vorgesehen sind.

Die erfindungsgemäßen Manganlegierungen sind so in ihrer Zusammensetzung zu wählen, dass sie biokorrodierbar sind. Als biokorrodierbar im Sinne der Erfindung werden Legierungen bezeichnet, bei denen in physiologischer Umgebung ein Abbau/Umbau stattfindet, so dass der aus dem Werkstoff bestehende Teil des Implantates ganz oder zumindest überwiegend nicht mehr vorhanden ist. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCl 0,4 g/l, MgSO₄ 0,1 g/l, NaHCO₃ 2,2 g/l, Na₂HPO₄ 0,126 g/l, NaH₂PO₄ 0,026 g/I). Eine Probe der zu untersuchenden Legierung wird dazu in einem verschlossenen Probenbehälter mit einer definierten Menge des Prüfmediums bei 37°C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Zur Einstellung der Zeit, in der das Implantat seine Funktionalität behalten soll, ist es von Vorteil, wenn die Wahl der speziellen Legierungszusammensetzung, mit ihrer speziellen Degradationskinetik und die Dimensionierung des Implantats aufeinander abgestimmt werden.

So ist es z.B. anzustreben, dass die mechanische Integrität eines Stents *in vivo* zwischen 2 und 6 Monaten stabil bleibt.

Anzumerken ist, dass Mangan für alle biologischen Organismen essentiell ist. Mangelerscheinungen beim Menschen sind jedoch nicht bekannt und selbst hohe Überdosen werden gut verkraftet. Der Tagesbedarf liegt bei 0,4 - 10 mg. Im Tierexperiment wurde Mangan als essentielles Element für die Knochen- und Knorpelbildung identifiziert. Weitere Tieruntersuchungen haben bei Mangan-Mangel Defizite in der Insulinproduktion gezeigt, Veränderungen im Lipoproteinstoffwechsel und Störungen im Metabolismus von Wachstumsfaktoren. Weiterhin ist Mangan möglicherweise ein notwendiger Kofaktor bei der Umwandlung von Präprothrombin zu Prothrombin. Biochemische Untersuchungen haben weiterhin gezeigt, dass Mangan einen Kofaktor für eine Reihe von Enzymen darstellt, unter anderem für die Arginase und die alkalische Phosphatase der Leber sowie für die Pyruvatcarboxylase. Durch Mangan wird auch die Aktivität der Succinatdehydrogenase und der Prolidase sowie einiger Enzyme der Mukopolysaccharidsynthese gesteigert. Dementsprechend ist die Freisetzung von geringen Mengen Mangan im Körper im Zuge der Degradation der Legierung toxikologisch unbedenklich.

Implantate im Sinne der Erfindung sind über ein chirurgisches oder minimalinvasives Verfahren in den Körper eingebrachte Vorrichtungen und umfassen Befestigungselemente für Knochen, beispielsweise Schrauben, Platten oder Nägel, chirurgisches Nahtmaterial, Darmklammern, Gefäßclips, Prothesen im Bereich des Hart- und Weichgewebes, beispielsweise Stents, und Ankerelemente für Elektroden, insbesondere von Schrittmachern oder Defibrillatoren. Das Implantat besteht ganz oder in Teilen aus dem biokorrodierbaren Werkstoff.

Vorzugsweise ist das Implantat aus der biokorrodierbaren Manganlegierung ein Stent für Blutgefäße, Gallengang, Harnröhre, Speiseröhre etc.; d. h. ein Stütz- oder Verbindungsimplantat für alle Gefäße, Gangsysteme oder Hohlraumverbindungen im menschlichen Körper. Stents herkömmlicher Bauart weisen eine filigrane Struktur aus metallischen Streben auf, die zur Einbringung in den Körper zunächst in einem nicht-expandierten Zustand vorliegt und die am Ort der Applikation dann in einen expandierten Zustand aufgeweitet wird.

Weiterhin ist das Implantat aus der biokorrodierbaren Manganlegierung insbesondere ein Clip zum Verschließen von abgetrennten Blutgefäßen. Zum Beispiel ein v-förmiger Clip, mit dem ein abgetrenntes Gefäß verschlossen wird, in dem der Clip mit einer Zange am Gefäßende so zusammengequetscht/plastisch deformiert wird, dass er das Gefäßende verschließt, so dass der Blutfluss zum Stillstand kommt und das Blut thrombosiert.

Außerdem kann das Implantat aus der biokorrodierbaren Manganlegierung insbesondere ein Implantat sein, das dazu verwendet wird, Gefäße, in die eine Kanüle oder ein Katheter mit größerem Durchmesser in das Gefäßsystem temporär eingeführt worden war, nach Entfernen des temporären Implantats, wieder sicher zu verschließen, um Blutungen an dieser Stelle zu vermeiden. In diesem Fall hat das Implantat typischerweise die Form einer Klammer, die mittels eines Applikationssystems implantiert wird und bei der Krallen oder Spitzen die Gefäßwand um die zu schließende Stelle herum greifen und die Öffnung zusammen pressen.

Schließlich ist das Implantat aus der biokorrodierbaren Manganlegierung insbesondere ein Okkluder, speziell ein Septumokkluder. Ein Okkluder ist ein minimalinvasiv applizierbares Fixierungssystem, mit dem z.B. ein Septumdefekt (PFO) so lange fixiert werden kann bis die Herzscheidewand verwachsen und der Defekt natürlich verschlossen wird. Danach kann das Implantat biologisch abgebaut werden. Dabei wird z. B. das Implantat so ausgeführt, dass ein langes röhrenförmiges Gebilde durch eine Zugkraft vor und hinter dem Defekt zusammengestaucht und plastisch verformt wird, so dass sich auf beiden Seiten des Defekts eine Schirmform ausbildet, die beide Teile der offenen Herzscheidewand zusammenpresst.

## Patentansprüche

1. Implantat mit einem Grundkörper bestehend ganz oder in Teilen aus einem biokorrodierbaren metallischen Werkstoff, **dadurch gekennzeichnet, dass** der Werkstoff eine Legierung ist, deren Hauptbestandteil Mangan ist, wobei als Hauptbestandteil die Legierungskomponente verstanden wird, deren Atomanteil an der Legierung am höchsten ist.

2. Implantat nach Anspruch 1, **dadurch gekennzeichnet, dass** der Werkstoff eine Legierung ist, deren Hauptbestandteil γ-Mangan ist.

3. Implantat nach Anspruch 1 oder 2, **dadurch gekennzeichnet, dass** der Werkstoff Eisen und/oder Iridium enthält.

4. Implantat nach Anspruch 3, bei dem der Anteil von Eisen an der Manganlegierung 0 bis kleiner 50% beträgt.

5. Implantat nach Anspruch 4, bei dem der Anteil von Eisen an der Manganlegierung 40 bis 49% beträgt.

6. Implantat nach Anspruch 4, bei dem der Anteil von Eisen an der Manganlegierung 5 bis 40% und bevorzugt 10 bis 30% beträgt.

7. Implantat nach Anspruch 3, bei dem der Anteil von Iridium an der Manganlegierung 0 bis 25% und bevorzugt 8 bis 15% beträgt.

8. Implantat nach Anspruch 3, bei dem der Anteil von Eisen an der Manganlegierung zwischen 30 bis 40% und der Anteil an Iridium an der Manganlegierung bis zu 10% beträgt.

9. Implantat nach Anspruch 3, bei dem der Anteil von Eisen an der Manganlegierung zwischen 10 bis 20% und der Anteil an Iridium an der Manganlegierung 8 bis 20% beträgt.

10. Implantat nach Anspruch 3, bei dem die Anteile von Eisen und Iridium an der Manganlegierung gemäß
Formel (I) Mn - Fe_{(50-3.57X)} - Ir_{X}, wobei X = 0.1 - 13.99,
betragen.

11. Implantat nach Anspruch 3, bei dem die Anteile von Eisen und Iridium an der Manganlegierung gemäß
Formel (II) Mn - Fe_{(40-5.0X)} - Ir_{X}, wobei mit X = 0.1 - 7.99,
betragen.

12. Implantat nach Anspruch 3, bei dem die Anteile von Eisen und Iridium an der Manganlegierung gemäß
Formel (III) Mn - Fe_{(50-2.0X)} - Ir_{X}, wobei X = 0.1 - 24.99,
betragen.

13. Implantat nach Anspruch 3, bei dem die Anteile von Eisen und Iridium an der Manganlegierung gemäß
Formel (IV) Mn - Fe_{(20-10.0X)} - Ir_{X}, wobei X = 0.1 - 1.99,
betragen.

14. Implantat nach einem der vorhergehenden Ansprüche, bei dem die Manganlegierung zusätzlich ein oder mehrere Elemente ausgewählt aus der Gruppe bestehend aus Kupfer, Gold, Palladium, Platin, Rhenium, Ruthenium, Vanadium, Kohlenstoff, Stickstoff, Arsen und Selen enthält.

15. Implantat nach einem der vorhergehenden Ansprüche, bei dem das Implantat ein Stent, ein Clip, ein Okkluder oder ein Implantat zur temporären Fixierung von Gewebe ist.

## Claims

1. An implant with a main body consisting entirely or in parts of a biocorrodible metallic material, **characterised in that** the material is an alloy of which the main component is manganese, wherein the alloy component of which the atomic proportion in the alloy is greatest is understood to be the main component.

2. The implant according to claim 1, **characterised in that** the material is an alloy of which the main component is γ-manganese.

3. The implant according to claim 1 or 2, **characterised in that** the material contains iron and/or iridium.

4. The implant according to claim 3, in which the proportion of iron in the manganese alloy is 0 to less than 50%.

5. The implant according to claim 4, in which the proportion of iron in the manganese alloy is 40 to 49%.

6. The implant according to claim 4, in which the proportion of iron in the manganese alloy is 5 to 40% and preferably 10 to 30%.

7. The implant according to claim 3, in which the proportion of iridium in the manganese alloy is 0 to 25% and preferably 8 to 15%.

8. The implant according to claim 3, in which the proportion of iron in the manganese alloy is between 30 and 40% and the proportion of iridium in the manganese alloy is up to 10%.

9. The implant according to claim 3, in which the proportion of iron in the manganese alloy is between 10 and 20% and the proportion of iridium in the manganese alloy is 8 to 20%.

10. The implant according to claim 3, in which the proportions of iron and iridium in the manganese alloy are according to
formula (I) Mn-Fe_{(50-3.57X)}-Ir_{X}, where X=0.1-13.99.

11. The implant according to claim 3, in which the proportions of iron and iridium in the manganese alloy are
according to formula (II) Mn-Fe_{(40-5.0X)}-Ir_{X}, where X=0.1-7.99.

12. The implant according to claim 3, in which the proportions of iron and iridium in the manganese alloy are
according to formula (III) Mn-Fe_{(50-2.0X})-Ir_{X}, where X=0.1-24.99. □□

13. The implant according to claim 3, in which the proportions of iron and iridium in the manganese alloy are
according to formula (IV) Mn-Fe_{(20-10.0X})-Ir_{X}, where X=0.1-1.99.

14. The implant according to any one of the preceding claims, in which the manganese alloy additionally contains one or more elements selected from the group comprising copper, gold, palladium, platinum, rhenium, ruthenium, vanadium, carbon, nitrogen, arsenic and selenium.

15. The implant according to any one of the preceding claims, in which the implant is a stent, a clip, an occluder or an implant for temporarily fixing tissue.

## Revendications

1. Implant avec un corps de base constitué totalement ou en partie d'un matériau métallique biocorrodable, **caractérisé en ce que** le matériau est un alliage dont le constituant principal est le manganèse, où en tant que constituant principal, il s'agit du composant de l'alliage dont la proportion en atomes est la plus élevée dans l'alliage.

2. Implant selon la revendication 1, **caractérisé en ce que** le matériau est un alliage dont le constituant principal est le manganèse γ.

3. Implant selon la revendication 1, ou 2, **caractérisé en ce que** le matériau contient du fer et/ou de l'iridium.

4. Implant selon la revendication 3, chez lequel la proportion de fer dans l'alliage de manganèse se situe de 0 à moins de 50 %.

5. Implant selon la revendication 4, chez lequel la proportion de fer dans l'alliage de manganèse se situe de 40 à 49 %.

6. Implant selon la revendication 4, chez lequel la proportion de fer dans l'alliage de manganèse se situe de 5 à 40 %, et de préférence de 10 à 30 %.

7. Implant selon la revendication 3, chez lequel la proportion d'iridium dans l'alliage de manganèse se situe de 0 à 25 %, et de préférence de 8 à 15 %.

8. Implant selon la revendication 3, chez lequel la proportion de fer dans l'alliage de manganèse se situe entre 30 et 40 % et la proportion d'iridium dans l'alliage de manganèse s'élève jusqu'à 10 %.

9. Implant selon la revendication 3, chez lequel la proportion de fer dans l'alliage de manganèse se situe de 10 à 20 % et la proportion d'iridium dans l'alliage de manganèse se situe de 8 à 20 %.

10. Implant selon la revendication 3, chez lequel les proportions de fer et d'iridium dans l'alliage de manganèse sont selon
la formule (I) Mn - Fe_{(50-3,57X)} - Ir_{X}, où X = 0,1 à 13,99.

11. Implant selon la revendication 3, chez lequel les proportions de fer et d'iridium dans l'alliage de manganèse sont selon
la formule (II) Mn - Fe_{(40-5,0X)} - Ir_{X}, où X = 0,1 à 7,99.

12. Implant selon la revendication 3, chez lequel les proportions de fer et d'iridium dans l'alliage de manganèse sont selon
la formule (III) Mn - Fe_{(50-2,0X}) - Ir_{X}, où X = 0,1 à 24,99.

13. Implant selon la revendication 3, chez lequel les proportions de fer et d'iridium dans l'alliage de manganèse sont selon
la formule (IV) Mn - Fe_{(20-10,0X)} - Ir_{X}, où X = 0,1 à 1,99.

14. Implant selon l'une des revendications précédentes, dans lequel l'alliage de manganèse contient en outre un ou plusieurs éléments choisis dans le groupe constitué du cuivre, de l'or, du palladium, du platine, du rhénium, du ruthénium, du vanadium, du carbone, de l'azote, de l'arsenic et du sélénium.

15. Implant selon l'une des revendications précédentes, dans lequel l'implant est un stent, un clip, un occludeur ou un implant pour la fixation temporaire de tissu.
